# EUROPEAN PATENT SPECIFICATION

(11) **EP 3 941 544 B1**
(45) Date of publication and mention of the grant of the patent: **02.07.2025**
(21) Application number: 20705344.8
(22) Date of filing: 13.02.2020
(51) Int. Cl.: A61L 29/08, A61L 29/14, A61L 31/08, A61L 31/14

(54) **MEDICAL DEVICE AND PROCESS OF PREPARING A MEDICAL DEVICE**
MEDIZINPRODUKT UND VERFAHREN ZUR HERSTELLUNG EINES MEDIZINPRODUKTS
DISPOSITIF MÉDICAL ET PROCÉDÉ DE PRÉPARATION D'UN DISPOSITIF MÉDICAL

(30) Priority: 19.03.2019 CH 3452019
(43) Date of publication of application: 26.01.2022
(73) Proprietor: Qvanteq AG, 8005 Zürich (CH)
(72) Inventor: BUZZI, Stefano, 8903 Birmensdorf (CH); MÄDER, Armin, 8805 Richterswil (CH); ZUCKER, Arik, 8003 Zürich (CH)
(74) Representative: Latscha Schöllhorn Partner AG
(86) International application number: PCT/EP2020/053670
(87) International publication number: WO 2020/187493

(56) References cited:
- EP-A1- 2 992 908
- WO-A1-2013/011305
- WO-A1-2018/154069
- WO-A1-2018/189185

## Description

### Technical Field

The present invention relates to a medical device to be applied to a body of a human or animal being according to the preamble of independent claim 1, to a method of preparing such a medical device and a medical device set.

Such medical devices comprising a contact surface to contact the body of the human or animal being, which contact surface is covered with a soluble surface sealing, can be used in a variety of medical applications. For example such medical devices can be implants to be permanently or temporarily set into the body, implantable devices or devices to be applied by temporary provision into the body.

### Background Art

In many medical treatments, devices are used which have to be applied to or into a body of a human or animal patient. In order to be compatible with the organism of the patient, i.e. biocompatible, or to prevent disturbance of the organism, contact surfaces of the medical devices are specifically prepared. For example, such contact surfaces can be cleaned or sterilized to prevent infections or transfer of contaminations into the body. To protect the prepared contact surfaces of medical devices it is known to provide appropriate packings configured to maintain the characteristics of the prepared contact surfaces.

Also, it is known to cover the contact surfaces of medical devices with protective layers. For example, in US 2009/0132048 A1 a dental implant is described, wherein its implant body is covered with a protective layer dissolving in contact with the bone it is to be set. The protective layer is made of a salt. By having the salty protective layer, the dental implant can be suitable to keep the properties of the outer surface of the implant body until it is set in the bone. Moreover, by being soluble the protective layer can be automatically removed while or shortly after being set into the bone. No additional specific step for removing the protective layer is required. EP 2 992 908 A1 describes a method for treating a surface of an implant.

However, even though such salty protective layers of the art provide for conveniently protecting the surface characteristics of the implant body until being set into the bone, they typically tend to brittle. Therefore, the dental implants have to be carefully handled prior being set and, particularly, it has to be prevented that the protective layer is mechanically strained. Furthermore, the salty protective layer is not suitable for reliably maintaining some specific characteristics of the surface. For example, in many applications it is desired to apply medical device with a contact surface being hydrated. However, the hydration itself or the composition of the hydration of the surface can be impaired by a salty protective layer.

Therefore, there is a need for a medical device having a contact surface which is protected, wherein the characteristics of the contact surface and particularly its hydration can safely be maintained.

### Disclosure of the Invention

According to the invention this need is settled by a medical device to be applied to a body of a human or animal being, as it is defined by the features of independent claim 1, a process of preparing a medical device, as defined by the features of independent claim 8, and by a set, as defined by the features of independent claim 15.

In particular, in one aspect the invention is a medical device to be applied to a body of a human or animal being. The medical device comprises a contact surface to contact the body of the human or animal being when the medical device is applied to the body of the human or animal being. The contact surface is covered with a soluble surface sealing, wherein the surface sealing is composed of a carbohydrate. The contact surface is configured to provide target characteristics comprising a functionalization obtained by the contact surface being provided with double or more charged phosphate ions such that the phosphate ions are exposed to a bodily fluid when the medical device is applied to the body of the human or animal being. The medical device is an implantable catheter or another catheter, a tubing, a pump or an impeller pump to forward a bodily fluid such as blood.

Thereby, the carbohydrate is preferably a di- or a trisaccharide. Preferably, the carbohydrate is Trehalose, Maltotriose, Lactose, Lactulose, Palatinose, or Sucrose. For example, Trehalose, has proven to be suitable for creating a surface sealing according to the present invention and also proves to be stable to elevated temperatures.

Alternatively, the carbohydrate preferably is a monosaccharide or a sugar alcohol, such as Threitol, Erythritol, Glucose, Fructose, Sorbitol, Galactose, Galactitol, Mannose, Mannitol, Xylitol, Myo-inositol or similar, organic acids such as citric acid, or other substances such as vitamin C.

In some applications, it can be sufficient to provide only the contact surface of the medical device with the surface sealing and to leave other surface portions of the medical device uncovered. However, it can also be beneficial to provide the essentially complete outer boundary of the medical device to be in contact with the body or a bodily fluid with the surface sealing. Like this, the complete contact surface can be efficiently covered and protected.

By covering the contact surface with the surface sealing, the characteristics of the contact surface can be preserved. In particular, surface characteristic which may be generated by preparing the contact surface can be maintained until the medical device is applied and the contact surface is provided to its target location. Thus, the surface sealing protects the contact surface and its characteristics prior application of the medical device. Furthermore, carbohydrates such as sugars are highly soluble such that the surface sealing can be removed automatically when or shortly after the medical device is located at its target application position such that the contact surface having its prepared specific characteristics or properties can efficiently be exposed. Preferably, the surface sealing is configured to dissolve within 30 seconds, within 20 seconds or within 10 seconds when being in contact with an aqueous solution such as a bodily fluid like blood or the like. Like this, the surface sealing can be automatically removed as soon as it comes into contact with the bodily fluid, a washing buffer or another liquid that is used for device flushing. When being applied to the body or during flushing prior to application it is advantageous to quickly dissolve the surface sealing such that the perfectly pure contact surface is exposed as directly upon application.

Furthermore, by being composed of a carbohydrate and particularly a sugar as mentioned above, the surface sealing may remain attached to the contact surface, even when it is comparably plain, without peeling off, even under some applied mechanical stress and/or after a prolonged time (i.e. aging). In fact, surface sealings of carbohydrates such as sugars have shown a pronounced elasticity and compliance to deformations. Like this, the surface sealing can be comparably little prone to brittle and crack when mechanical stressed. Also, it can be applied to comparably complex geometries, e.g. with undercuts or the like, as well as complex surfaces, e.g. which bear a certain roughness that may also include undercuts.

Still further, surface sealings of carbohydrates or sugars have shown a surprisingly advantageous capability to stably preserve target characteristics provided to contact surfaces. In particular, such surface sealings have shown to be capable of maintaining a hydration of the contact surface. Such hydration is beneficial and therefore a key feature in many applications of medical devices such as when the device is implanted and in contact with blood or other bodily fluid or into bone.

Also, the surface sealing composed of the carbohydrate forms a cover shielding the contact surface from an interaction with contaminating agents which is stable under comparably hot and cold conditions. The carbohydrate additionally allows for providing the surface sealing stable in comparably high humidity and during sterilization of the complete medical device.

Preferably, the surface sealing is configured to withstand all or commonly used sterilization processes or techniques. Thereby, the sterilization preferably comprises gamma radiation, e-beam provision, or ethylene oxide provision. Such sterilization allows for preparing the medical device including its surface sealing to be applied to the body and particularly to be set into the body.

The thickness of the surface sealing covering the contact surface can be in a range of up to few hundred micrometer. The surface sealing can take the form of a film thin enough to advantageously allow movements and flexibility. The thickness can particularly be thinner than the structures it coats.

Preferably, the surface sealing is homogeneous. In particular, the surface sealing can be homogeneous by being more or less uniformly distributed over the contact surface. Thereby, the surface sealing can still comprise plural different components such as different saccharides or sugars as mentioned above. Such homogeneous surface sealing allows for being uniformly and rapidly dissolved and removed.

Preferably, the surface sealing is seamlessly covering the contact surface. Like this, the contact surface can be uniformly covered and contamination can continuously be prevented.

Preferably, the surface sealing is gas-tight. Such surface sealing allows for efficiently preventing contamination of the surface through the atmosphere. Like this, an organic surface sealing that prevents organic atmospheric contamination can be provided.

The medical device is an implantable catheter or another catheter, a tubing, a pump, or an impeller pump to forward a bodily fluid such as blood.

In embodiments not covered by the invention the medical device is an implant such as an artificial joint or the like, a dental implant or an abutment. In such embodiments the implant preferably is configured to replace a natural biological structure of the body of the human or animal being, or such implant can be a bone plate, a bone screw, a heart valve, a vascular stent, a part of a cardiac pace maker such as an electrode, a cochlear implantable hearing aid or the like.

The implant of such embodiments not covered by the invention preferably is essentially rigid. The term "essentially rigid" in this connection relates to reduced or non-flexibility of an outer boundary or dimension of the medical device. Thereby, different portions of the medical device still may be movable relative to each other, but the shape of the single portions stays during such movement. Also, portions inside the implant may be flexible or elastic but still covered or embedded in a rigid housing. In connection with the essentially rigid implants the surface sealing composed of the organic compound can be particularly beneficial.

The surface sealing preferably is configured to be dissolved when the implant is implanted at a target location. In particular, the surface sealing preferably is configured to be dissolved when arriving at the target location or before being implanted at the target location. Thereby, it can be configured in accordance with a predefined insertion procedure such that it can be assured that the surface sealing is dissolved when finally implanting it. The term "implanting" as used in this connection can relate to securing the implant at or in the body lumen at the target location. Such a surface sealing allows for preserving original mechanical properties of the implant even though its surface is protected by the surface sealing before and eventually while inserting it.

Preferably, the surface sealing dissolves shortly before, during or after insertion in the body lumen, for instance upon contact with the bodily fluid passing through a body lumen, bone or other structure. Thus, the function of shielding the defined contact surface characteristics remains effective at least from applying the surface sealing during production throughout packaging, sterilization, storage, transport and unpacking. As soon as the implant is prepared and ready to be inserted into the body, the function of shielding the implant's surface is not necessary anymore and the surface sealing can be dissolved at this time or at least shortly after insertion of the implant in the body. The implant received in, or on, the given body lumen is consequently in its best condition to ensure success of treatment.

Preferably, at least a portion of the medical device forming the contact surface is made of a metal, a metal alloy, a ceramic material, glass, a metal oxide, or a polymeric material. For example, said portion of the medical device can be made of titanium or apatite. Implants as mentioned above can also be made of titanium alloy such as Nitinol, cobalt chrome alloys, platinum chrome alloys, or stainless steel.

In one preferred embodiment, the contact surface is a plain surface. The term "plain" as associated with possible embodiments of the implants according to the present invention can indicate a substantially smooth surface whose roughness is comprised in a range of up to 10 micrometer or advantageously up to 5 micrometer. Such a plain surface also poses special requirements with respect to the material of the surface sealing, which must be able to adhere to such a plain surface.

Thereby, the plain surface preferably lacks a substantial roughness or waviness of its topology or any substantial texture or a coating or a combination thereof. Such a plain surface may also lack - or may have been purposely deprived of - any substantial roughness, or at least any roughness at an interior surface of the implant, or waviness of its topology or any substantial texture or a coating of any kind. This may prove especially advantageous for supporting a thorough cleaning or purification of the medical device and, conversely, for preventing contamination from the environment such as ambient atmosphere, from storage means or from manipulation. In this sense, the preferred, optional absence of specific treatments for integrating a complicated roughness or peak and valley topography in the medical device may prove beneficial to making it less susceptible to contamination.

In another preferred embodiments, the contact surface is a roughened surface.

According to the claimed invention, the contact surface is configured to provide target characteristics, as is defined in the independent claims. Thereby, the target characteristics may comprise a hydration. By providing the contact surface with hydration the target surface can be embodied hydrophilic. In particular, the contact surface may have a comparably high hydrophilicity which may be an essential antithrombotic characteristic and/or an essential biocompatible characteristic, and which is to be preserved by the surface sealing. Thus, by having the hydrated contact surface covered and protected by the surface sealing it can be achieved that the hydration is present until the implant is applied to the body at its target location.

The surface characteristics of the contact surface according to the claimed invention are embodied by a functionalization e.g., implemented by providing exposition of charged ions to a bodily fluid. In particular, according to the claimed invention, the target characteristics of the contact surface comprises a functionalization obtained by the contact surface being provided with double or more charged ions such that the ions are exposed to a bodily fluid when the medical device is applied to the body of the human or animal being, e.g. by being inserted in a body lumen. The ions used according to the claimed invention are phosphate ions. Additionally, such ions can be anions comprising sulfate, borate or carbonate groups or organic acids, any combination thereof, or molecules with more than one charged group.

By providing double or more charged ions to the contact surface of the medical device and thereby establishing the functionalized surface, the risk of thrombus formation that can lead to restenosis can be essentially reduced and acceptance of the medical device by the body can be essentially increased. It has been shown, that by exposing the ions, instead of a bare metal surface, to the body lumen or, particularly, a bodily fluid circulating therein, thrombogenicity can be significantly reduced. Thus, provision of the double or more charged ions allows for functionalizing the contact surface.

Furthermore, such provision of double or more charged ions to the contact surface of the medical device may achieve a comparably strong and solid functionalization of the contact surface. More specifically, such functionalization can be particularly resistant to mechanical stress. Therefore, it can be especially beneficial when being applied to moving medical devices. For example, implantable impeller pumps may rotate at a velocity of more than 1'000 revolutions per minute (rpm) such as at about 5'000 rpm or even up to 20'000 rpm or higher. For achieving a resistant functionalization to provide low risk of thrombus formation in components moving that fast, the provision of double or more charged ions can be specifically beneficial in particular because, when in the human body, the antithrombotic surface can be the result of an equilibrium of ions on the surface and in the surrounding body lumen.

Preferably, the surface sealing comprises a combination of at least two different carbohydrates such as sugars or saccharides. Thereby, it may be particularly advantageous if the different carbohydrates are different fast dissolving sugars such as monosaccharides, disaccharides, trisaccharides or polymeric sugars. For example, such combination can comprise two different monosaccharides, a monosaccharide and a disaccharide, a monosaccharide and a trisaccharide, a monosaccharide and a polymeric sugar, two different disaccharides, a disaccharide and a trisaccharide, a disaccharide and a polymeric sugar, two different trisaccharides, two different polymeric sugars or a trisaccharide and a polymeric sugar. Therefore, the surface sealing preferably comprises a combination of at least a monosaccharide such as Glucose and a disaccharide such as Trehalose. Thereby, the surface sealing preferably comprises the monosaccharide at a relative concentration of about 50% or more. Also combinations of more than two different saccharides or sugars may be beneficial. Optionally, the combination is supplemented with other compounds such as a polymer or a salt.

In context of the composition or configuration of the surface sealing, surface characteristics, surface topology, roughness and flexibility of the medical device together represent a complex system that can react differently in environments. More specifically, parameters influenced, e.g., by transportation, storage, sterilization etc. such as humidity, temperature or radiation may affect the protection of the target characteristics such as hydration by the surface sealing. The composition of the surface sealing being a variable in the overall configuration can have an impact on the preservation of the target characteristics of the contact surface. While it can be advantageous for some configurations to use single type of sugars in the sealing, it has been shown that other configurations benefit from a combination of at least two or more different sugars in the sealing. Thereby, the relative and absolute concentration of the sealing components is relevant too. While the preservation of a particular target characteristic on a given medical device may be well preserved with one particular surface sealing, a different target characteristic on the same medical device may require a different sealing.

For example, while a monosaccharide such as Glucose for example works well to protect a surface characteristics functionalized with ions, the same sealant does typically not provide adequate protection for target characteristics including hydration and consequently hydrophilicity. Further, disaccharides such as Trehalose have shown to be effective in protecting target characteristics functionalized with phosphate ions. Combinations of monosaccharides such as Glucose and a disaccharide such as Trehalose, in particular sealants with a relative concentration of monosaccharide of about 50% and more, have shown to be preferred in protecting certain hydrophilic target characteristics and can also be favourable in protecting target characteristics functionalized with ions such as phosphate.

In another aspect, the invention is a process of preparing a medical device. The process comprises the steps of: (i) obtaining a medical device being an implantable catheter or another catheter, a tubing, a pump, or an impeller pump to forward a bodily fluid such as blood, wherein the medical device has a contact surface configured to contact a body of a human or animal being, (ii) submerging the contact surface in an aqueous solution, providing the contact surface with double or more charged phosphate ions such that the phosphate ions are exposed to a bodily fluid when the medical device is applied to the body of the human or animal being, (iii) providing a sealing agent comprising a carbohydrate into the aqueous solution, (iv) removing the contact surface from the aqueous solution, and (v) drying the contact surface such that the dried sealing agent forms a surface sealing covering the contact surface.

The process according to the invention allows for cleaning and hydrating the contact surface in one step together with providing the surface sealing to the contact surface. Like this, a hydrated and protected contact surface can very efficiently be manufactured.

Further, by the process according to the invention the surface sealing can efficiently be formed uniformly and consistently over the contact surface in a way that no region meant to be sealed is left uncovered or is scaled. This can efficiently be achieved by applying the sealing agent in the aqueous solution, for example, between 1% to 10% and, preferably, in a solution between 1% to 6%.

When drying the sealing agent on the contact surface, the medical device can be arranged in a package such as in the insertion device or a part thereof. Thereby, the sealing agent can bind or merge the medical device to the package such that that package and medical device form a unit.

In one preferred embodiment, the contact surface is submerged in the aqueous solution prior the sealing agent being provided into the aqueous solution. Like this, it can be assured that the contact surface is sufficiently hydrated before it is covered by the surface sealing. In particular, the time the contact surface is exposed to the aqueous solution without the sealing agent can be adjusted as desired.

In another preferred embodiment, the contact surface is submerged in the aqueous solution after the sealing agent being provided into the aqueous solution. Such all in one step allows for a particular efficient preparation of the contact surface.

The term "sealing agent" can relate to a single or plural substances which form the surface sealing on the contact surface of the medical device. In particular, it can be a substance solved in a liquid and forming the surface sealing when being dried.

The method comprises a step of providing the contact surface with double or more charged phosphate ions such that the ions are exposed to a bodily fluid when the medical device is applied to the body of the human or animal being. Such step advantageously is performed prior to submerging the contact surface in the aqueous solution.

Further ions may comprise sulfate, borate or carbonate groups or organic acids, any combination thereof, or molecules with more than one charged group. The ions are Phosphate ions.

As described in more detail above in connection with the medical device according to the invention and its preferred embodiments, providing the contact surface with double or more charged ions can be particularly beneficial.

The sealing agent comprises a carbohydrate and preferably a di- or a trisaccharide or Trehalose, Maltotriose, Lactose, Lactulose, Palatinose, or Sucrose. Such sealing agent allows to provide a surface sealing as described above. Alternatively, the carbohydrate can be a monosaccharide or a sugar alcohol, such as Threitol, Erythritol, Glucose, Fructose, Sorbitol, Galactose, Galactitol, Mannose, Mannitol, Xylitol, Myo-inositol or similar, organic acids such as citric acid, or other substances such as vitamin C.

A sealing agent made of sugar can be advantageously stable and elastic to an appropriate extent. It has been found that, e.g., Trehalose is especially stable, both when undergoing mechanical and thermal stresses, and suitable to maintain formerly created specific contact surface characteristics unaltered.

Preferably, the sealing agent comprises a combination of at least two different carbohydrates. Thereby, the sealing agent advantageously comprises a combination of at least a monosaccharide such as Glucose and a disaccharide such as Trehalose, wherein the sealing agent preferably comprises the monosaccharide at a relative concentration of about 50% or more. When providing the contact surface with double or more charged ions and the hydration, such sealing agent allows for providing the beneficial effects described in more detail above in connection with the respective preferred embodiment of the medical device according to the invention.

Preferably, the contact surface is treated to remove contaminants prior being submerged into the aqueous solution. Such pre-treatment allows for providing a particular appropriate contact surface.

Preferably, the process further comprises a step of sterilizing the medical device after drying the contact surface. Such sterilizing step allows for providing the medical device ready to use. Sterilization can be performed by applying a radiation or a gas. To this purpose, gamma or beta radiations and/or a gaseous sterilisation agent can be used, such as ethylene oxide (ETO) or similar.

In a further other aspect, the invention is a set comprising a medical device as described above and a package configured to protect the contact surface of the medical device. Thereby, the package preferably is configured to maintain the contact surface of the medical device in a dry condition.

Such package can further reinforce protection from shocks or other mechanical/thermal stresses, scratches etc. to the surface sealing, ultimately contributing to the preservation of the target characteristics imparted to the contact surface. The package can be made of a polymer, or another material commonly used for packaging, or a combination thereof. It can comprise a cover for opening and/or closing the package. Furthermore, the cover can be equipped with a pierceable structure such as a septum or the like. Such a structure allows for providing a medium such as a solvent such as a saline solution into the package, e.g., via a needle such as by means of a syringe or the like without opening it. The package allows for maintaining the integrity of the contact surface.

As opposed to prior art solutions which rely on liquid substances in the container, the package according to the present invention is configured to store the medical device in a dry condition that is without use of a liquid or gel-like storage means.

The set of the present invention can further comprise a pouch, configured to receive the medical device and the package for storage. The pouch is preferably permeable to a gaseous sterilising agent and/or radiation sterilisable. Thus, the above described step of sterilisation can be executed through such especially modified pouch.

When using such a pouch the set preferably is, after sterilization, packed in a further bag, envelope, sheath or the like. Like this, it can be achieved that after sterilisation and during long-term storage, inner content of the permeable pouch can be protected from, e.g., moisture.

### Brief Description of the Drawings

The medical device according to the invention, as well as set according to the invention are described in more detail herein below by way of a reference embodiment and with reference to the attached drawings. This reference embodiment and the corresponding drawings relate to a bone implant, and not to a device according to the claimed invention. This embodiment is provided for illustrative purposes only.
- Fig. 1: shows a schematic cross sectional view of a bone implant as a reference-embodiment of a medical device (not according to the claimed invention);
- Fig. 2: shows a schematic cross sectional view of the bone implant of Fig. 1, wherein a contact surface is hydrated;
- Fig. 3: shows a schematic cross sectional view of the bone implant of Fig. 2, wherein the contact surface together with its hydration is covered by a surface sealing; and
- Fig. 4: shows a schematic cross sectional view of the bone implant of Fig. 3 when being applied to a body of a patient by implantation into a jaw bone of the patient.

### Description of Embodiments

In the following description certain terms are used for reasons of convenience and are not intended to limit the invention. The terms "right", "left", "up", "down", "under" and "above" refer to directions in the figures. The terminology comprises the explicitly mentioned terms as well as their derivations and terms with a similar meaning. Also, spatially relative terms, such as "beneath", "below", "lower", "above", "upper", "proximal", "distal", and the like, may be used to describe one element's or feature's relationship to another element or feature as illustrated in the figures. These spatially relative terms are intended to encompass different positions and orientations of the devices in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. The devices may be otherwise oriented (rotated 90 degrees or at other orientations), and the spatially relative descriptors used herein interpreted accordingly. Likewise, descriptions of movement along and around various axes include various special device positions and orientations.

To avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

As explained above, given the relevant influence of contact surfaces of medical devices on critical aspects of healing or biological acceptance, such as inflammatory and hyperplastic responses, it has been found that preventively assigning certain target surface characteristics to the medical devices, before application, is advantageous.

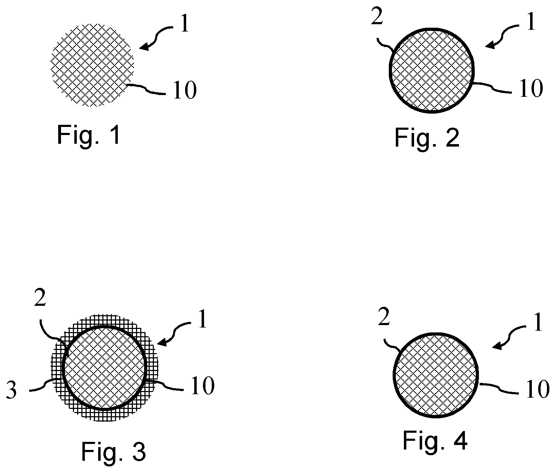

Fig. 1 shows a cross sectional view of a bone implant 1 as a reference-embodiment of a medical device (bone implants are not according to the claimed invention). It is to be understood that, even though the invention is exemplified by means of a bone implant, which is not according to the claimed invention, it is applicable to a variety of other medical devices as well, i.e. to an implantable catheter, another catheter, a tubing, a pump, or an impeller pump to forward a bodily fluid such as blood, which are according to the claimed invention.

The circumference of an implantable body of the bone implant 1 forms a contact surface 10. Typically, the implantable body of the bone implant 1 is provided with a thread (not visible in the Figs.) to be screwed into a jaw bone. The bone implant 1 is made of titanium. The contact surface 10 is roughened to allow efficient grow-in into the bone tissue after the implant being set into the jaw bone. In Fig. 1 the bone implant 1 is shown after manufactured where the contact surface is cleaned and pre-processed.

For preparing the bone implant 1, the contact surface is submerged in a bath of an aqueous solution. Thereby, as can be seen in Fig. 2, a hydration 2 is provided to the contact surface 10. Then, Trehalose or a combination of at least two different carbohydrates is provided as sealing agent into the aqueous solution such that the aqueous solution has a predefined concentration of Trehalose or each of the different carbohydrates. The Trehalose or combination of different carbohydrates adheres at the hydration 2 and stabilizes it. Then the contact surface 10 is removed from the aqueous solution and dried.

In Fig. 3, the bone implant 1 is shown after drying the contact surface 10. It can be seen that a surface sealing 3 consisting of Trehalose or the combination of different carbohydrates is generated. The surface sealing 3 completely covers the hydration 2 of the contact surface 10. The surface sealing 3 protects the contact surface 10 including its hydration 2. In particular, it allows to maintain the hydration 2 until the bone implant 1 is set into the jaw bone.

Fig. 4 shows the bone implant 1 when being applied. In particular, the surface sealing 3 is dissolved by an aqueous solution and like this removed from the contact surface 10 and hydration 2. In one possible application, the bone implant 1 is flushed by the aqueous solution prior to setting into the jaw bone. Like this, the surface sealing 3 can efficiently be removed shortly before being set. In another possible application, the bone implant 1 is set together with its surface sealing 3. The surface sealing 3 is then dissolved while setting the implant by blood or another fluid as the aqueous solution. Like this, the surface sealing 3 can efficiently be removed in one step while setting the implant without requiring any previous preparation.

In any case, as can be seen in Fig. 4, at the end of the dissolving the surface sealing 3, the hydration 2 still is on the contact surface 10. When being applied or implanted, the hydration 2 and its preferred properties are exposed to the bone tissue.

In addition to maintaining the hydration 2 on the contact surface 10 up to the implant being applied, the surface sealing 3 allows to maintain the hydration 2 and the contact surface 10 clean. Like this, it can be prevented that preventively engineered surface characteristics are compromised by environment or other contaminants, such as hydrocarbon deposits or deposits of other undesired organic matter, machining impurities, fibers, dust or the like. All such contaminants would be located on the surface sealing 3 and removed together with it.

The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

## Claims

1. A medical device to be applied to a body of a human or animal being, comprising
a contact surface to contact the body of the human or animal being when the medical device is applied to the body of the human or animal being, wherein
the contact surface is covered with a soluble surface sealing,
**characterized in that**
the surface sealing is composed of a carbohydrate,
the medical device is an implantable catheter or another catheter, a tubing, a pump, or an impeller pump to forward a bodily fluid such as blood, and
the contact surface is configured to provide target characteristics comprising a functionalization obtained by the contact surface being provided with double or more charged Phosphate ions such that the Phosphate ions are exposed to a bodily fluid when the medical device is applied to the body of the human or animal being.

2. The medical device of claim 1, wherein the carbohydrate is a di- or a trisaccharide, wherein the carbohydrate preferably is Trehalose, Maltotriose, Lactose, Lactulose, Palatinose, or Sucrose.

3. The medical device of claim 1, wherein the carbohydrate is a monosaccharide or a sugar alcohol, such as Threitol, Erythritol, Glucose, Fructose, Sorbitol, Galactose, Galactitol, Mannose, Mannitol, Xylitol, Myo-inositol, organic acids such as citric acid, or vitamin C.

4. The medical device of any one of the preceding claims, wherein the surface sealing
comprises a combination of at least two different carbohydrates, and/or
is configured to withstand sterilization preferably comprising gamma radiation, e-beam provision, or ethylene oxide provision, and/or
is configured to dissolve within 30 seconds when being in contact with an aqueous solution, and/or
is homogeneous, and/or
is seamlessly covering the contact surface and/or
is gas-tight.

5. The medical device of any one of the preceding claims, wherein at least a portion of the medical device forming the contact surface is made of a metal, a metal alloy, a ceramic material, glass, a metal oxide, or a polymeric material.

6. The medical device of any one of the preceding claims, wherein the contact surface is a plain surface, preferably lacking a substantial roughness or waviness of its topology or any substantial texture or a coating or a combination thereof.

7. The medical device of any one of the preceding claims, wherein the target characteristics comprises a hydration, wherein the surface sealing preferably comprises a combination of at least a monosaccharide such as Glucose and a disaccharide such as Trehalose, wherein the surface sealing preferably comprises the monosaccharide at a relative concentration of about 50% or more.

8. A process of preparing a medical device, comprising
obtaining a medical device being an implantable catheter or another catheter, a tubing, a pump, an impeller pump to forward a bodily fluid such as blood, wherein the medical device has a contact surface configured to contact a body of a human or animal being,
submerging the contact surface in an aqueous solution,
providing the contact surface with double or more charged Phosphate ions such that the Phosphate ions are exposed to a bodily fluid when the medical device is applied to the body of the human or animal being,
providing a sealing agent comprising a carbohydrate into the aqueous solution,
removing the contact surface from the aqueous solution, and
drying the contact surface to form a surface sealing covering the contact surface.

9. The process of claim 8, wherein the contact surface is submerged in the aqueous solution prior the sealing agent being provided into the aqueous solution, or wherein the contact surface is submerged in the aqueous solution after the sealing agent being provided into the aqueous solution.

10. The process of claim 8 or 9, wherein the carbohydrate is a di- or a trisaccharide, and/or the carbohydrate is Trehalose, Maltotriose, Lactose, Lactulose, Palatinose, or Sucrose.

11. The process of claim 8 or 9, wherein the carbohydrate is a monosaccharide or a sugar alcohol, such as Threitol, Erythritol, Glucose, Fructose, Sorbitol, Galactose, Galactitol, Mannose, Mannitol, Xylitol, Myo-inositol, organic acids such as citric acid, or vitamin C.

12. The process of any one of claims 8 to 11, wherein the sealing agent comprises a combination of at least two different carbohydrates, wherein the sealing agent preferably comprises a combination of at least a monosaccharide such as Glucose and a disaccharide such as Trehalose, wherein the sealing agent preferably comprises the monosaccharide at a relative concentration of about 50% or more.

13. The process of any one of claims 8 to 12, wherein the contact surface is treated to remove contaminants prior being submerged into the aqueous solution.

14. The process of any one claims 8 to 13, comprising sterilizing the medical device after drying the contact surface.

15. A set comprising a medical device according to any one of claims 1 to 7 and a package configured to protect the contact surface of the medical device, wherein the package preferably is configured to maintain the contact surface of the medical device in a dry condition.

## Patentansprüche

1. Medizinische Vorrichtung, um an einem Körper eines Menschen oder Tiers angewendet zu werden, umfassend
eine Kontaktoberfläche, um den Körper des Menschen oder Tiers zu berühren, wenn die medizinische Vorrichtung an dem Körper des Menschen oder Tiers angewendet wird, wobei
die Kontaktoberfläche mit einer löslichen Oberflächenversiegelung bedeckt ist, **dadurch gekennzeichnet, dass** die Oberflächenversiegelung aus einem Kohlenhydrat besteht, die medizinische Vorrichtung ein implantierbarer Katheter oder ein anderer Katheter, ein Schlauch, eine Pumpe, oder eine Flügelradpumpe ist, um ein Körperfluid, wie Blut, zu befördern, und
die Kontaktoberfläche konfiguriert ist, um Zieleigenschaften bereitzustellen, umfassend eine Funktionalisierung, die dadurch erhalten wird, dass die Kontaktoberfläche mit doppelt oder mehrfach geladenen Phosphationen derart versehen wird, dass die Phosphationen einem Körperfluid ausgesetzt werden, wenn die medizinische Vorrichtung an dem Körper des Menschen oder Tiers angewendet wird.

2. Medizinische Vorrichtung nach Anspruch 1, wobei das Kohlenhydrat ein Di- oder Trisaccharid ist, wobei das Kohlenhydrat vorzugsweise Trehalose, Maltotriose, Lactose, Lactulose, Palatinose oder Saccharose ist.

3. Medizinische Vorrichtung nach Anspruch 1, wobei das Kohlenhydrat ein Monosaccharid oder ein Zuckeralkohol, wie Threitol, Erythritol, Glucose, Fructose, Sorbitol, Galactose, Galactitol, Mannose, Mannitol, Xylitol, Myo-Inositol, organische Säuren wie Zitronensäure oder Vitamin C, ist.

4. Medizinische Vorrichtung nach einem der vorstehenden Ansprüche, wobei die Oberflächenversiegelung
eine Kombination aus mindestens zwei unterschiedlichen Kohlenhydraten umfasst, und/oder
konfiguriert ist, um eine Sterilisation auszuhalten, vorzugsweise umfassend Gammabestrahlung, Elektronenstrahlbereitstellung oder Ethylenoxidbereitstellung, und/oder
konfiguriert ist, um sich bei einem Kontakt mit einer wässrigen Lösung innerhalb von 30 Sekunden aufzulösen, und/oder
homogen ist, und/oder
die Kontaktoberfläche nahtlos abdeckt,und/oder
gasdicht ist.

5. Medizinische Vorrichtung nach einem der vorstehenden Ansprüche, wobei mindestens ein Abschnitt der medizinischen Vorrichtung, die die Kontaktoberfläche bildet, aus einem Metall, einer Metalllegierung, einem keramischen Material, Glas, einem Metalloxid oder einem Polymermaterial hergestellt ist.

6. Medizinische Vorrichtung nach einem der vorstehenden Ansprüche, wobei die Kontaktoberfläche eine glatte Oberfläche ist, deren Topologie vorzugsweise eine wesentliche Rauheit oder Welligkeit oder eine beliebige wesentliche Textur oder Beschichtung oder eine Kombination davon fehlt.

7. Medizinische Vorrichtung nach einem der vorstehenden Ansprüche, wobei die Zieleigenschaften eine Hydratisierung umfassen, wobei die Oberflächenversiegelung vorzugsweise eine Kombination aus mindestens einem Monosaccharid wie Glucose, und einem Disaccharid, wie Trehalose, umfasst, wobei die Oberflächenversiegelung vorzugsweise das Monosaccharid in einer relativen Konzentration von etwa 50 % oder mehr umfasst.

8. Verfahren zum Herstellen einer medizinischen Vorrichtung, umfassend
Erhalten einer medizinischen Vorrichtung, die ein implantierbarer Katheter oder ein anderer Katheter, ein Schlauch, eine Pumpe oder eine Flügelradpumpe ist, um ein Körperfluid, wie Blut, zu befördern, wobei die medizinische Vorrichtung eine Kontaktoberfläche aufweist, die konfiguriert ist, um einen Körper eines Menschen oder eines Tiers zu berühren, Eintauchen der Kontaktoberfläche in eine wässrige Lösung,
Versehen der Kontaktoberfläche mit doppelt oder mehrfach geladenen Phosphationen derart, dass die Phosphationen einem Körperfluid ausgesetzt werden, wenn die medizinische Vorrichtung auf den Körper des Menschen oder Tiers angewendet wird,
Bereitstellen eines Versiegelungsmittels, umfassend ein Kohlenhydrat, in die wässrige Lösung,
Entfernen der Kontaktoberfläche aus der wässrigen Lösung und Trocknen der Kontaktoberfläche, um eine Oberflächenversiegelung auszubilden, die die Kontaktoberfläche abdeckt.

9. Verfahren nach Anspruch 8, wobei die Kontaktoberfläche in die wässrige Lösung eingetaucht wird, bevor das Versiegelungsmittel in die wässrige Lösung bereitgestellt wird, oder wobei die Kontaktoberfläche in die wässrige Lösung eingetaucht wird, nachdem das Versiegelungsmittel in die wässrige Lösung bereitgestellt wurde.

10. Verfahren nach Anspruch 8 oder 9, wobei das Kohlenhydrat ein Di- oder Trisaccharid ist und/oder das Kohlenhydrat Trehalose, Maltotriose, Lactose, Lactulose, Palatinose oder Saccharose ist.

11. Verfahren nach Anspruch 8 oder 9, wobei das Kohlenhydrat ein Monosaccharid oder ein Zuckeralkohol, wie Threitol, Erythritol, Glucose, Fructose, Sorbitol, Galactose, Galactitol, Mannose, Mannitol, Xylitol, Myo-Inositol, organische Säuren wie Zitronensäure oder Vitamin C ist.

12. Verfahren nach einem der Ansprüche 8 bis 11, wobei das Versiegelungsmittel eine Kombination aus mindestens zwei unterschiedlichen Kohlenhydraten umfasst, wobei das Versiegelungsmittel vorzugsweise eine Kombination aus mindestens einem Monosaccharid, wie Glucose, und einem Disaccharid, wie Trehalose, umfasst, wobei das Versiegelungsmittel das Monosaccharid vorzugsweise in einer relativen Konzentration von etwa 50 % oder mehr umfasst.

13. Verfahren nach einem der Ansprüche 8 bis 12, wobei die Kontaktoberfläche behandelt wird, um Verunreinigungen vor dem Eintauchen in die wässrige Lösung zu entfernen.

14. Verfahren nach einem der Ansprüche 8 bis 13, umfassend ein Sterilisieren der medizinischen Vorrichtung nach dem Trocknen der Kontaktoberfläche.

15. Satz, umfassend eine medizinische Vorrichtung nach einem der Ansprüche 1 bis 7 und eine Verpackung, die konfiguriert ist, um die Kontaktoberfläche der medizinischen Vorrichtung zu schützen, wobei die Verpackung vorzugsweise konfiguriert ist, um die Kontaktoberfläche der medizinischen Vorrichtung in einem trockenen Zustand zu halten.

## Revendications

1. Dispositif médical destiné à être appliqué sur un corps d'un être humain ou d'un animal, comprenant
une surface de contact pour entrer en contact avec le corps de l'être humain ou de l'animal lorsque le dispositif médical est appliqué sur le corps de l'être humain ou de l'animal, dans lequel
la surface de contact est recouverte d'une étanchéité de surface soluble,
**caractérisé en ce que**
l'étanchéité de surface est constituée d'un hydrate de carbone,
le dispositif médical est un cathéter implantable ou un autre cathéter, une tubulure, une pompe, ou une turbopompe pour acheminer un fluide corporel tel que le sang, et
la surface de contact est conçue pour fournir des caractéristiques cibles comprenant une fonctionnalisation obtenue en dotant la surface de contact d'ions phosphate doublement chargés ou plus de telle sorte que les ions phosphate sont exposés à un fluide corporel lorsque le dispositif médical est appliqué sur le corps de l'être humain ou de l'animal.

2. Dispositif médical selon la revendication 1, dans lequel l'hydrate de carbone est un di- ou un trisaccharide, dans lequel l'hydrate de carbone est de préférence le tréhalose, le maltotriose, le lactose, le lactulose, le palatinose ou le saccharose.

3. Dispositif médical selon la revendication 1, dans lequel l'hydrate de carbone est un monosaccharide ou un alcool de sucre, tel que le thréitol, l'érythritol, le glucose, le fructose, le sorbitol, le galactose, le galactitol, le mannose, le mannitol, le xylitol, le myo-inositol, des acides organiques tels que l'acide citrique, ou la vitamine C.

4. Dispositif médical selon l'une quelconque des revendications précédentes, dans lequel l'étanchéité de surface
comprend une combinaison d'au moins deux hydrates de carbone différents, et/ou
est conçue pour supporter une stérilisation comprenant de préférence un rayonnement gamma, un apport de faisceau d'électrons ou un apport d'oxyde d'éthylène, et/ou
est conçue pour se dissoudre en 30 secondes au contact d'une solution aqueuse, et/ou
est homogène, et/ou
recouvre sans discontinuité la surface de contact et/ou
est étanche au gaz.

5. Dispositif médical selon l'une quelconque des revendications précédentes, dans lequel au moins une partie du dispositif médical formant la surface de contact est composée d'un métal, d'un alliage métallique, d'un matériau céramique, de verre, d'un oxyde métallique ou d'un matériau polymère.

6. Dispositif médical selon l'une quelconque des revendications précédentes, dans lequel la surface de contact est une surface lisse, de préférence dépourvue d'une rugosité ou d'une ondulation notable de sa topologie ou d'une quelconque texture notable ou d'un revêtement ou d'une combinaison de ceux-ci.

7. Dispositif médical selon l'une quelconque des revendications précédentes, dans lequel les caractéristiques cibles comprennent une hydratation, dans lequel l'étanchéité de surface comprend de préférence une combinaison d'au moins un monosaccharide tel que le glucose et d'un disaccharide tel que le tréhalose, dans lequel l'étanchéité de surface comprend de préférence le monosaccharide à une concentration relative égale ou supérieure à environ 50 %.

8. Procédé de préparation d'un dispositif médical, comprenant
l'obtention d'un dispositif médical étant un cathéter implantable ou un autre cathéter, une tubulure, une pompe, une turbopompe pour acheminer un fluide corporel tel que le sang, dans lequel le dispositif médical a une surface de contact conçue pour entrer en contact avec un corps d'un être humain ou d'un animal,
l'immersion de la surface de contact dans une solution aqueuse,
le fait de doter la surface de contact d'ions phosphate doublement chargés ou plus de telle sorte que les ions phosphate sont exposés à un fluide corporel lorsque le dispositif médical est appliqué sur le corps de l'être humain ou de l'animal,
la fourniture d'un agent d'étanchéité comprenant un hydrate de carbone dans la solution aqueuse,
le retrait de la surface de contact de la solution aqueuse, et
le séchage de la surface de contact pour former une étanchéité de surface recouvrant la surface de contact.

9. Procédé selon la revendication 8, dans lequel la surface de contact est immergée dans la solution aqueuse avant que l'agent d'étanchéité ne soit introduit dans la solution aqueuse, ou dans lequel la surface de contact est immergée dans la solution aqueuse après que l'agent d'étanchéité a été introduit dans la solution aqueuse.

10. Procédé selon la revendication 8 ou 9, dans lequel l'hydrate de carbone est un di- ou trisaccharide, et/ou l'hydrate de carbone est le tréhalose, le maltotriose, le lactose, le lactulose, le palatinose ou le saccharose.

11. Procédé selon la revendication 8 ou 9, dans lequel l'hydrate de carbone est un monosaccharide ou un alcool de sucre, tel que le thréitol, l'érythritol, le glucose, le fructose, le sorbitol, le galactose, le galactitol, le mannose, le mannitol, le xylitol, le myo-inositol, des acides organiques tels que l'acide citrique, ou la vitamine C.

12. Procédé selon l'une quelconque des revendications 8 à 11, dans lequel l'agent d'étanchéité comprend une combinaison d'au moins deux hydrates de carbone différents, dans lequel l'agent d'étanchéité comprend de préférence une combinaison d'au moins un monosaccharide tel que le glucose et d'un disaccharide tel que le tréhalose, dans lequel l'agent d'étanchéité comprend de préférence le monosaccharide à une concentration relative égale ou supérieure à environ 50 %.

13. Procédé selon l'une quelconque des revendications 8 à 12, dans lequel la surface de contact est traitée pour éliminer des contaminants avant d'être immergée dans la solution aqueuse.

14. Procédé selon l'une quelconque des revendications 8 à 13, comprenant la stérilisation du dispositif médical après le séchage de la surface de contact.

15. Ensemble comprenant un dispositif médical selon l'une quelconque des revendications 1 à 7 et un emballage conçu pour protéger la surface de contact du dispositif médical, dans lequel l'emballage est de préférence conçu pour maintenir la surface de contact du dispositif médical à l'état sec.
